Europäisches Patentamt

European Patent Office

Office européen des brevets

(11) Publication number: **0 157 359**
A2

# EUROPEAN PATENT APPLICATION

(21) Application number: 85103663.2

(22) Date of filing: 27.03.85

(51) Int. Cl.⁴: **A 61 K 37/12**

(30) Priority: 02.04.84 US 595669

(43) Date of publication of application: 09.10.85
Bulletin 85/41

(84) Designated Contracting States: **AT BE CH DE FR GB IT
LI LU NL SE**

(71) Applicant: **National Jewish Hospital and Research
Center, 3800 East Colfax Avenue, Denver
Colorado 80206 (US)**

(72) Inventor: **Clark, Richard A., 8469 Lightning View Drive,
Parker Colorado 80134 (US)**
Inventor: **Piez, Karl A., 1120 Bay Laurel Drive, Menlo Park
California 94025 (US)**

(74) Representative: **Patentanwälte Schulze Horn und
Hoffmeister, Goldstrasse 36, D-4400 Münster (DE)**

(54) Collagen matrix for tissue repair.

(57) A biological matrix is provided comprising a non-immu-
nogenic atelopeptide collagen. The biological matrix is useful as a closure for gaping wounds since it allows angiogenesis and fibroplasia while being incorporated into the
wound be re-epithelialization.

In a medical treatment a gaping wound is packed with
the atelopeptide collagen and kept sterile while the epidermal layer migrates over the matrix and incorporates it into
the normal tissue.

ACTORUM AG

# COLLAGEN MATRIX FOR TISSUE REPAIR

This invention concerns a non-immunogenic collagen product useful as closure forgaping wounds. The collagen product when inserted into the woundallows angiogenesis and fiberplasia and is incorporated into the wound by re-epithelialization. An effective biological matrix for closure of wounds is thus provided.

## BACKGROUND

Collagen, a structural fibrous protein naturally found in connective tissue such as skin can be made into products which are non-immunogenic and are thus useful in treating certain damaged human tissue. Collagen has been used, for example as an injectable implant material for augmenting soft tissue (U.S. Patent Nos. 3,949,073 and 4,404,208). Collagen products are also useful dressings for shallow wounds such as burns where the epidermal and dermal layers of the skin are damaged (U.S. Patent Nos. 4,089,333 and 4,215,693). Deep wounds however, those for example caused by chemosurgery, pressure sores or physical trauma, involve damage or excision of tissues below the skin. Most deep wounds will eventually close normally by forming an epidermal layer over the damaged or excised tissue area. However, a deformed skin covering results. Some gaping wounds cannot be closed at all because large tissue deficits exist. A gaping wound could be closed and the configuration of the superficial skin could be restored if the damaged or excised tissue could be replaced. A biocompatible material such as a non-immunogenic collagen product having the characteristics of human tissue would be such a suitable replacement. However, heretofor such materials have been found to be unsuitable either because they do not form a proper matrix for angiogenesis and fibroplasia or because they arerejected as foreign substances by migrating epithelial cells rather than being incorporated into the wound. Accordingly, suitable biological matrices for gaping wounds have been sought.

## SUMMARY

The present invention provides a biological matrix comprising a non-immunogenic atelopeptide collagen. The biological matrix is useful as a closure for gaping wounds since it allows angiogenesis and fibroplasia while being incorporated into the wound by re-epithelialization.

In the method of the present invention, a gaping wound is packed with the atelopeptide collagen and kept sterile while the epidermal layer migrates over the matrix and incorporates it into the normal tissue.

## DETAILS

Collagen is obtained from the connective tissue of a wide variety of domesticated animals. In its naturally occuring state the fibrous collagen protein contains characteristic amino acid sequences, called Atelopeptides, which appear to be responsible for its immunogenicity. In order to be useful in the treatment of humans, these telopeptides must be removed form the native collagen. Patents concerned with forming atelopeptide collagen are U.S. Patent nos. 3,034,852; 3,121,049; 3,131,170; 3,314,861; 3,530,037; and 3,949,073. Atelopeptide collagen, particularly that prepared from bovine skin, is the subject of the present invention.

Most particularly the present invention is concerned with Zyderm (Collagen Corp., Palo Alto, California) which is described in U.S. Patent No. 3,949,073. Zyderm is prepared from bovine skin and is composed of reconstituted atelopeptide collagen in saline with a small amount of local anesthetic. The atelopeptide collagen material described in U.S. Patent No. 4,424,208 is also suitable for use in the method of the present invention.

It has been discovered that when atelopeptide collagen is packed into a gaping wound and maintained under sterile conditions that it forms a suitable support matrix for the new blood vessels and fibrous tissue which form as the wound heals. The collagen product becomes a supportive, non-absorbable tissue substitute which is pliable and non-immunogenic. Moreover, the atelopeptide collagen is incorporated into the wound. Re-generating epithelial cells grow in layers over the external surface of the collagen which incorporates the matrix within the healed tissue.

The following example is intended to illustrate this invention without limiting the same in any manner.

EXAMPLE

On the flanks of depilated guinea pigs, two 4mm punch biopsies were performed. One wound on each animal was packed with approximately 0.5cc of 35 mg/ml bovine skin collagen (Zyderm) and both wounds were covered by a semi-permeable adhesive dressing (Op-Site). At 3, 7 and 14 days tje wpimds were excised, fixed, and embedded in plastic. One micron thick section were cut and stained with Giemsa.

Although no difference in the rate of wound healing was noted between collagen packed and control wounds, in 5/6 wounds packed with collagen, the re-epithelializing epidermis migrated over the collagen thus incorporating it into the wound. In addition, neovascularization and ingrowth of fibroblasts had occurred in the implanted calf skin collagen by 7 days. By 14 days after extirpation both control and collagen packed wounds were completely re-epithelialized and the collagen implants had been completely vascularized and partially remodeled by the ingrown fibroblasts.

What is Claimed:

Use of atelopeptide collagen in a composition or as a medicament being incorporated into a gaping wound for effecting repair and aiding in healing of said wound.